# EUROPEAN PATENT APPLICATION

(11) **EP 0 998 924 A1**
(43) Date of publication of application: **10.05.2000**
(21) Application number: 98933888.4
(22) Date of filing: 22.07.1998
(51) Int. Cl.: A61K 31/05, A61K 31/34

(54) **ANTITUMOR AGENTS**

(30) Priority: 22.07.1997 JP 19577897
(71) Applicant: MEIJI MILK PRODUCTS COMPANY LIMITED, Tokyo 104-0031 (JP)
(72) Inventor: NOZAKI, Hiroshi, Okayama-shi, Okayama 703-8254 (JP); IINUMA, Munekazu, Gifu-shi, Gifu 500-8368 (JP); YAMADA, Masashi, Meiji Milk Products Co., Ltd.,, Sumida-ku, Tokyo 130-0021 (JP); SUMA, Yukie, Meiji Milk Products Co., Ltd.,, Sumida-ku, Tokyo 130-0021 (JP)
(74) Representative: Hartz, Nikolai F., Dr.
(86) International application number: JP9803273
(87) International publication number: WO9904776

(57) **Abstract**

Antitumor agents containing as the active ingredient a compound selected from among pallidol, miyabenol A, miyabenol C, α-viniferin, kobophenol A, 4,4-(1,3-butadiene-1,4-diyl)bisphenol, leachianol A, leachianol B, leachianol F, leachianol G, hopeaphenol and davidiol A and salts thereof. Because of having excellent topoisomerase II inhibitory effects, these compounds are usable as antitumor agents.

## Description

### Technical Field

The present invention relates to an antitumor agent containing a plant-derived compound exhibiting topoisomerase II inhibitory activity.

### Background Art

The active cycle of cells appearing during division and proliferation of cells strongly relates to the regulation of proliferation and differentiation of cells; i.e., the cell cycle. Therefore, elucidation of the regulation mechanism leads to elucidation of molecular mechanisms in pathological and physiological phenomena, including cancer, aging, and cell death. In addition, elucidation of the regulation mechanism is expected to provide important clues toward development of pharmaceutical drugs such as antitumor agents targeting the cell cycle.

The action mechanisms of a variety of conventionally developed antitumor agents have been studied, and as a result, it has been clarified that most of the agents exhibit specific inhibitory effects to the cell cycle. For example, antimetabolites such as 5-fluorouracil, 6-mercaptopurine, and methotrexate inhibit essential precursors for DNA synthesis, and the cell cycle terminates at the S phase. Alkaloids such as plant-derived vinplastine and vincristine, colchicine analogues such as colcemid, and mold-derived lysoxine have an inhibition point at the M phase. Adriamycin, etoposide, eryptecin, and the like act on topoisomerase II, and inhibit distribution of replicated DNA (the M phase). Camptothecin derivative CPT-11, which is a plant alkaloid, inhibits topoisomerase I and terminates the cell cycle at the G2 phase.

However, these conventional antitumor agents are not fully satisfactory, and further novel antitumor agents are demanded.

An object of the present invention is to provide a novel antitumor agent exhibiting topoisomerase II inhibitory activity.

### Disclosure of the Invention

In view of the foregoing, the present inventors have performed extensive studies, and have found that particular compounds derived from cyperaceous and leguminous plants or salts thereof have excellent topoisomerase II inhibitory activity and are effective as antitumor agents. The present invention has been accomplished on the basis of this finding.

Accordingly, the present invention provides an antitumor agent comprising, as an active ingredient, a compound selected from among pallidol, miyabenol A, miyabenol C, α-viniferin, kobophenol A, 4,4-(1,3-butadiene-1,4-diyl)bis-phenol, leachianol A, leachianol B, leachianol F, leachianol G, hopeaphenol, and davidiol A, or a salt thereof.

The present invention also provides an antitumor agent composition comprising the aforementioned compound or a salt thereof, and a pharmaceutically acceptable carrier.

The present invention also provides use of the aforementioned compound or salts thereof for producing an antitumor agent.

The present invention also provides a method for treating tumors, which comprises administering the aforementioned compound or salts thereof to a patient in need thereof.

### Best Modes for Carrying Out the Invention

The aforementioned compounds used in the antitumor agent of the present invention are known to be contained in plants, but are not known to exhibit topoisomerase II inhibitory activity and antitumor activity.

The aforementioned compounds can be obtained from cyperaceous and leguminous plants through extraction and isolation. Examples of cyperaceous plants which may be used include kobresia, maltifolia, morrowii, foliosissima, thunbergii, dispalata, reinii, and ciliato-morginata. Examples of leguminous plants include sophora. No particular limitation is imposed on the portion of these plants for extraction, and one or more portions selected from among fruit, pericarp, bark, root skin, leaf, root, and the like (hereinafter such portions will be referred to as "source material") may be used.

No particular limitation is imposed on the method for extraction, and the aforementioned compounds can be extracted from these source materials by use of a solvent, and isolated. For example, a source material is cut into fractions, dried, and ground. The thus-ground substances are subjected to extraction by use of an organic solvent such as benzene, ethyl acetate, methanol, or acetone, and the solvent is removed under reduced pressure, to thereby obtain an extract. The thus-obtained extract may be purified through recrystallization and silica gel column chromatography, to thereby isolate a component. In the present invention, an extract obtained at any step in the above procedure; for example, at the extraction step, may be used, so long as the extract contains the effective amount of one or more of the aforementioned compounds.

The chemical structures of the thus-obtained active ingredients of the present invention are described below.

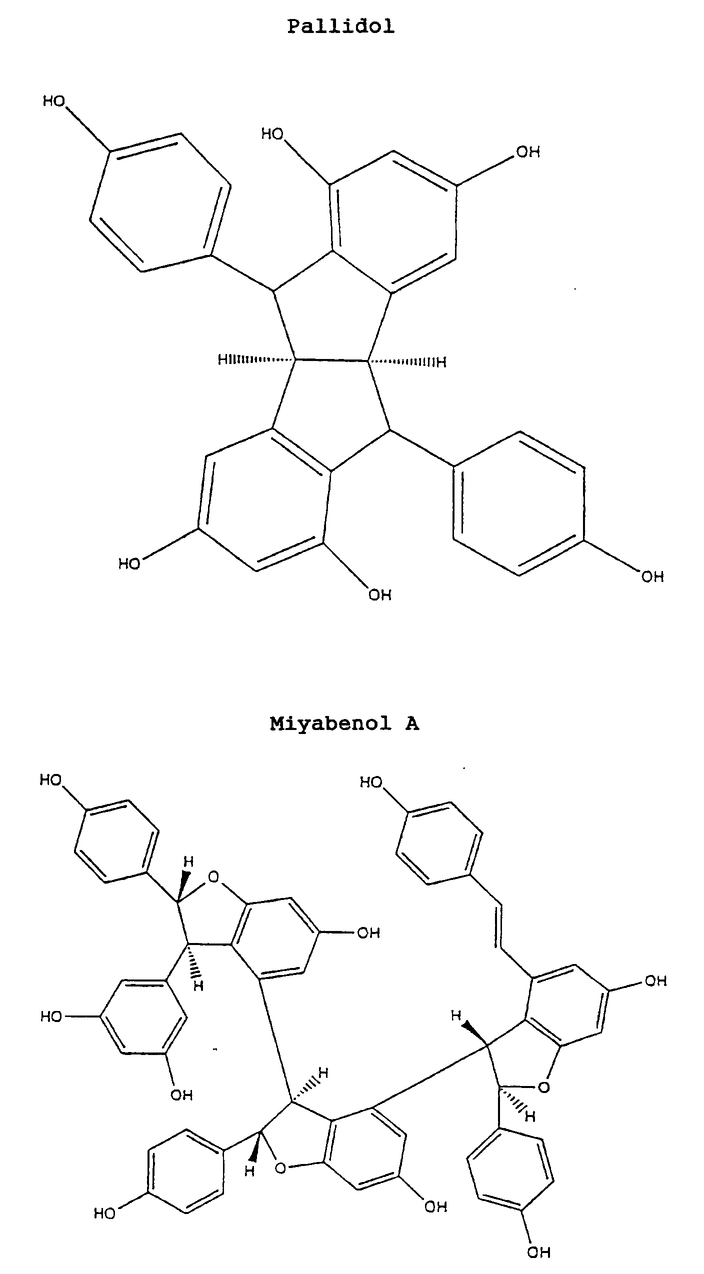

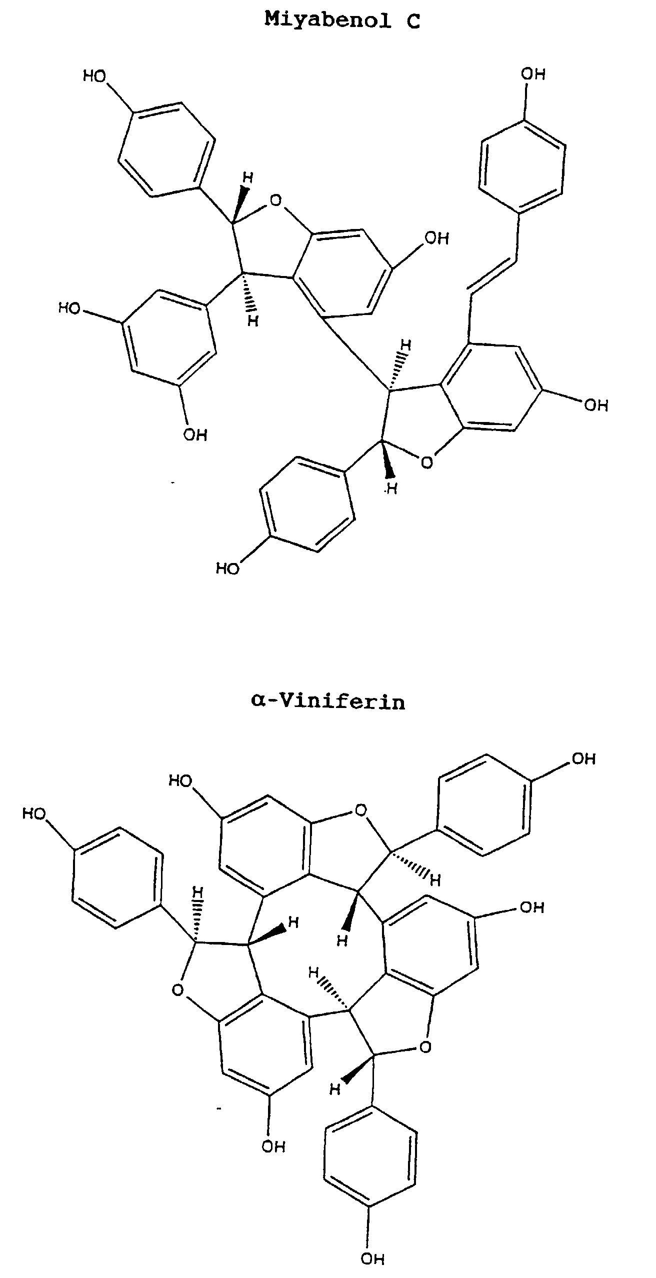

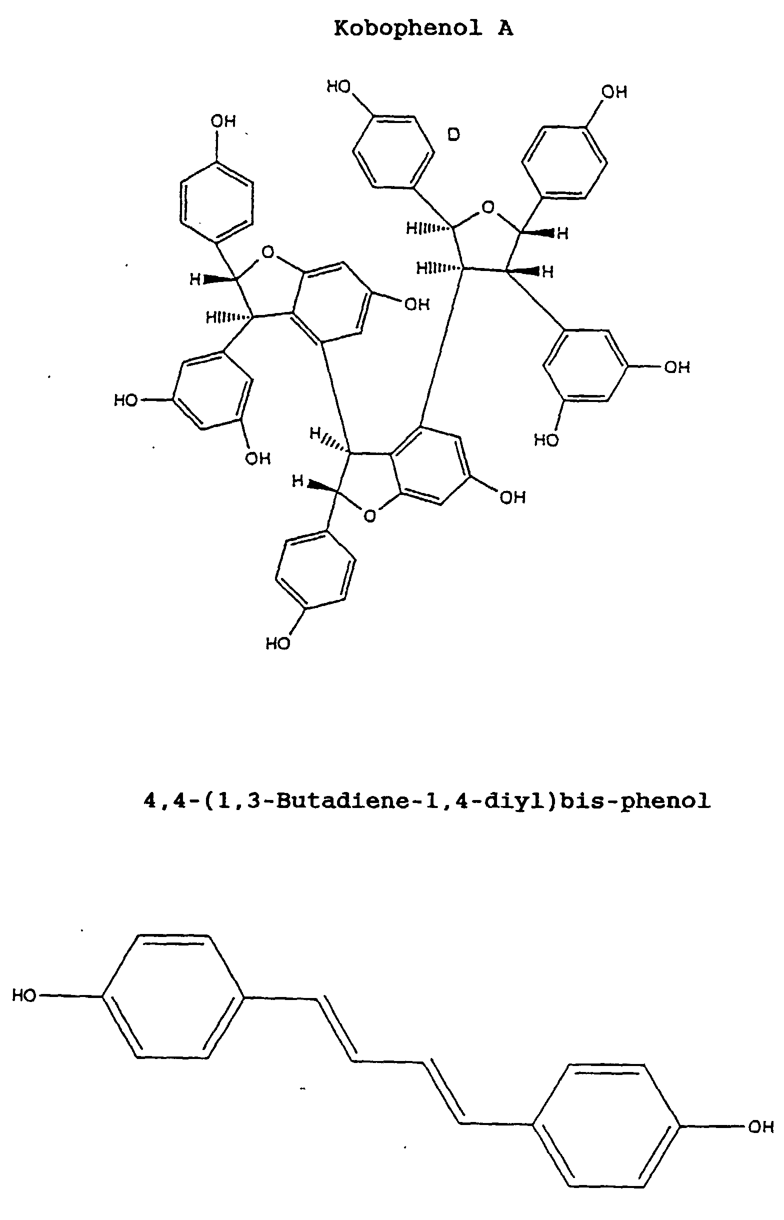

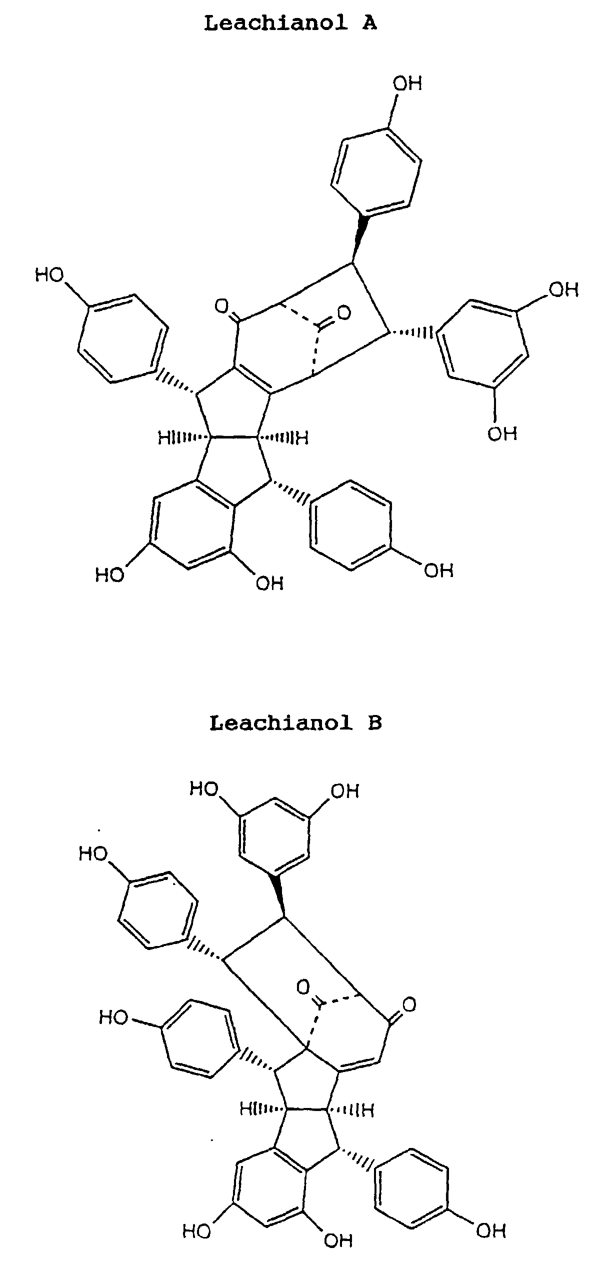

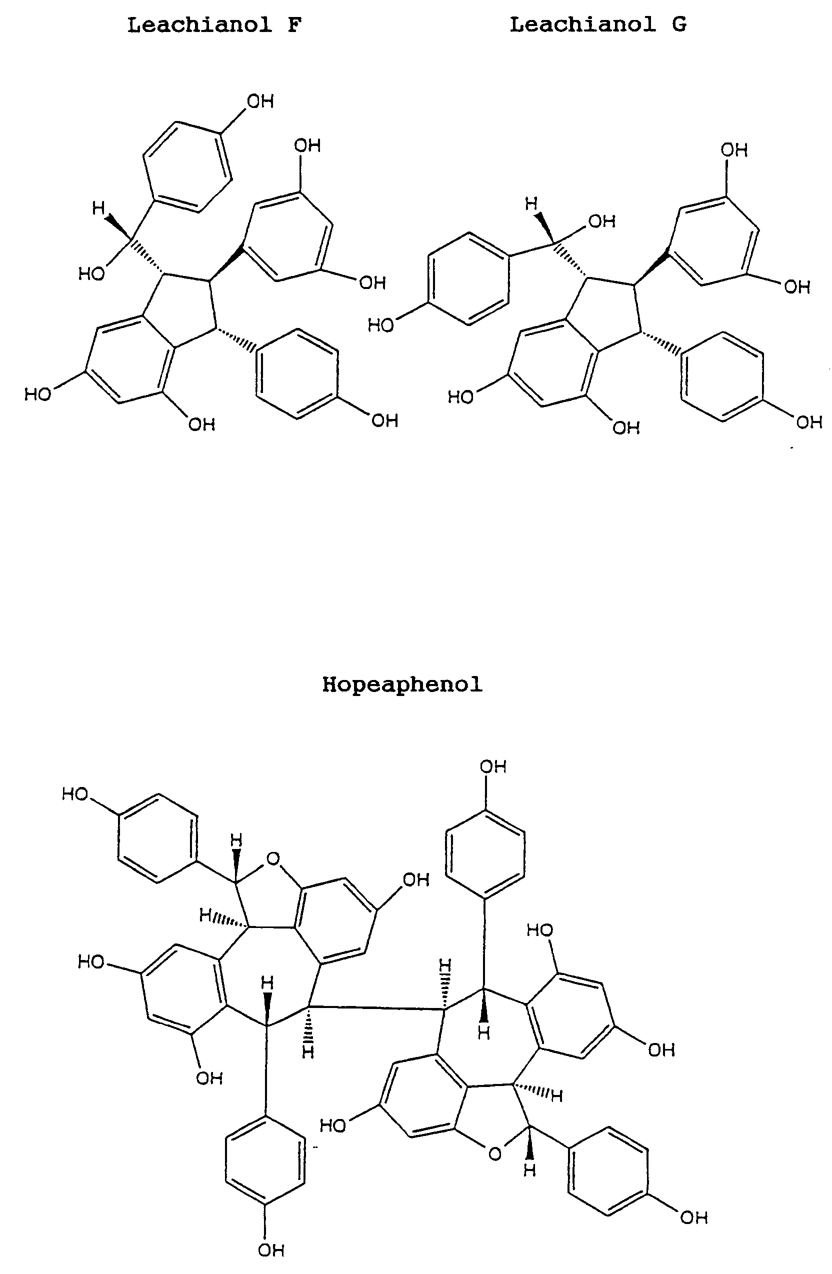

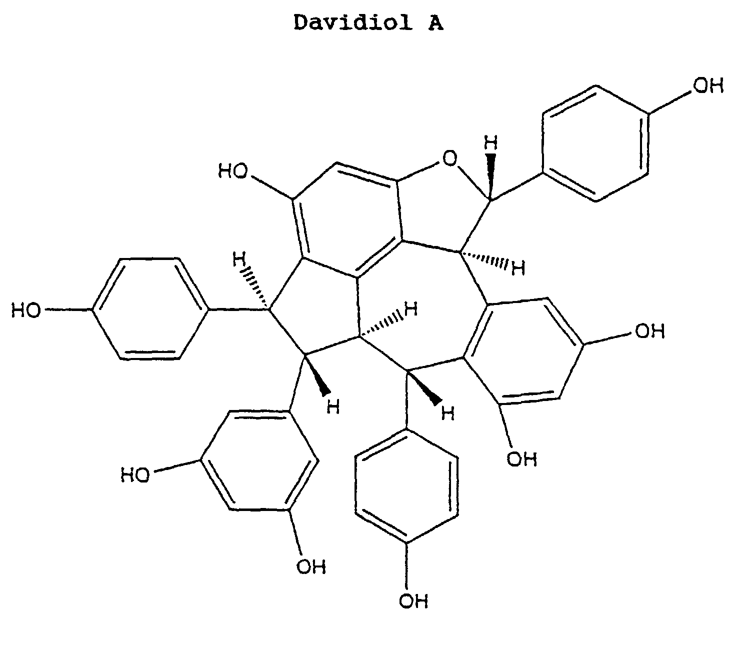

Examples of salts of the aforementioned compounds include hydrochlorides, hydrobromides, sulfates, perchlorates and other inorganic acid salts; sodium salts; and potassium salts. Of these, sodium salts are preferable in the present invention. The present invention also encompasses hydrates and solvates of the compounds or salts thereof.

The aforementioned compounds or salts exhibit excellent topoisomerase II inhibitory activity, and thus they are useful as antitumor agents.

The antitumor agent of the present invention can be administered orally or parenterally (e.g., intramuscularly, subcutaneously, intravenously, or as suppository). In the case of administration, the aforementioned compounds or salts thereof may contain pharmaceutically acceptable carriers, to thereby prepare antitumor agent compositions.

In the case of preparation of products for oral administration, the compounds may contain excipients, and if necessary, binders, disintegrators, lubricants, coloring agents, and sweetening and flavoring agents, to thereby prepare tablets, coated tablets, granules, capsules, solution agents, syrups, elixirs, oily or aqueous suspension agents by means of conventional techniques. Examples of excipients include lactose, cornstarch, white sugar, glucose, sorbitol, and crystal cellulose. Examples of binders include polyvinyl alcohol, polyvinyl ether, ethyl cellulose, methyl cellulose, gum arabic, tragacanth, gelatin, shellac, hydroxypropyl cellulose, hydroxypropyl starch, and polyvinyl pyrrolidone.

Examples of disintegrators include starch, agar, gelatin powder, crystal cellulose, calcium carbonate, sodium hydrogencarbonate, calcium citrate, dextran, and pectin. Examples of lubricants include magnesium stearate, talc, polyethylene glycol, silica, and hydrogenated vegetable oil. Coloring agents may be any pharmaceutically acceptable ones. Examples of sweetening and flavoring agents include cocoa powder, menthol, aromatic acids, peppermint oil, borneol, and cinnamon powder. If necessary, tablets or granules may optionally be coated by use of sugar coating, gelatin coating, or similar coating.

In the case of preparation of injection agents, if necessary, the compounds may contain pH-regulating agents, buffers, stabilizers, and preservatives, to thereby prepare agents for subcutaneous injection, intramuscular injection, and intravenous injection. Liquid injection agents may be stored in a container and freeze-dried, to thereby provide solid products, and the solid products may be prepared into injections upon use. Incidentally, a single dosage of the agent may be stored in a container, or a plurality of dosages may be stored in the same container.

In the case of administration to a human, a dosage of the antitumor agent of the present invention is usually 0.01-1,000 mg per adult per day, preferably 0.1-100 mg. In the case of administration to an animal, a dosage of the antitumor agent is usually 0.001-1,000 mg per kg body weight of the animal of interest per day, preferably 0.1-100 mg. The aforementioned dosage per day is administered in a single portion once a day, or in divided portions 2-4 times a day.

### Examples

The present invention will next be described by way of examples, which should not be construed as limiting the invention thereto.

### Example 1

Kobresia of Cyperaceae was washed with water, air-dried, and ground, and the ground kobresia was extracted with methanol. Methanol was removed from the resultant extract under reduced pressure, and the resultant residue was fractionated into an n-hexane-soluble portion, an ethyl acetate-soluble portion, and a butanol-soluble portion. The thus-obtained ethyl acetate-soluble portion was subjected to silica gel column chromatography wherein the column was filled with Kiesel Gel 60 (product of Merck). Thereafter, the portion was further subjected to ODS middle-pressure column chromatography and fractionation column chromatography, to thereby obtain pallidol (43 mg).

Pallidol (103.4 mg, 22 mg, and 1.1 mg) was obtained from thunbergii, dispalata, and reinii, respectively.

### Yellowish brown powder

Melting point: > 300°C
[α]_{D}=-84.59° (c=0.4 MeOH)
UVλₘₐₓ 281.4, 201.2nm(MeOH)
IRνₘₐₓ 3566-3160, 1610, 1468, 1258, 838cm⁻¹(KBr)
¹H-NMR(400MHz, acetone-d₆)δ : 3.82(2H,brs), 4.57(2H,brs), 6.20(2H,d,J=1.9Hz), 6.63(2H,d,J=1.8Hz), 6.71(4H,d,J=8.5Hz), 6.99(4H,d,J=8.5Hz)
¹³C-NMR(100MHz, acetone-d₆)δ : 53.9, 60.4, 102.4, 103.2, 115.7, 123.2, 129.0, 137.7, 150.2, 155.2, 156.2, 159.2
EIMS m/s : 454

### Example 2

In the same manner as in Example 1, miyabenol C (200 mg and 6.1 mg) was obtained from kobresia and morrowii, respectively.

### Yellowish brown powder

Melting point: 189-191°C
[α]_{D}=+69.28° (c=0.1 MeOH)
UVλₘₐₓ 296.2. 323.2nm(MeOH)
IRνₘₐₓ 3400, 1610, 1515, 1455cm⁻¹(KBr)
¹H-NMR(400MHz, acetone-d₆)δ : 4.29(1H,s), 4.63(1H,d,J=5.0Hz), 5.18(1H,brs), 5.37(1H,d,J=5.1Hz), 6.05(1H,d,J=2.2Hz). 6.15(1H,d,J=1.8Hz), 6.21(1H,t,J=2.2Hz), 6.28(1H,d,J=2.2Hz), 6.33(1H,d,J=1.8Hz), 6.47(2H,d,J=8.8Hz), 6.54(2H,d,J=8.4Hz), 6.58(1H,d,J=16Hz), 6.65(1H,s), 6.76(2H,d,J=8.8Hz), 6.85(2H,d,J=8.4Hz), 6.86(1H,d,J=16Hz), 7.09(2H,d,J=8.4Hz), 7.15(2H,d,J=8.8Hz)
¹³C-NMR(100MHz, acetone-d₆)δ : 50.3, 57.8, 92.9, 95.1, 97.2, 97.7, 103.1, 104.9, 107.5, 108.2, 116.2, 117.0, 117.3, 119.2, 122.4, 123.3, 128.0, 128.2, 129.2, 131.7, 133.9, 136.9, 144.5, 148.3, 158.2, 158.9, 159.0, 159.2, 160.8, 160.9, 162.3, 162.6

### Example 3

In the same manner as in Example 1, hopeaphenol (1,200 mg and 30.4 mg) was obtained from kobresia and thunbergii, respectively.

### Yellowish brown powder

Melting point: > 350°C
[α]_{D}=-70° (c=0.1 MeOH)
UVλₘₐₓ 284.8nm(MeOH)
IRνₘₐₓ 3250, 1610, 1520, 1447cm⁻¹(KBr)
¹H-NMR(400MHz, acetone-d₆)δ : 3.93(2H,s), 4.23(2H,d,J=12,1Hz), 5.17(2H,d,J=2.2Hz), 5.76(2H,d,J=12.1Hz), 5.80(2H,s), 6.29(2H,s), 6.54(2H,s), 6.56(4H,d,J=8.7Hz), 6.78(4H,d,J=8.7Hz), 6.91(4H,d,J=8.3Hz),7.13(4H,d,J=8.5Hz)
¹³C-NMR(100MHz, acetone-d₆)δ : 41.7, 48.8, 50.3, 88.7, 95.8, 101.7, 106.9, 111.8, 115.7, 116.5, 119.1, 121.7, 129.8, 130.8, 131.6, 135.8, 141.0, 143.0, 156.1, 157.7, 157.8, 159.0, 159.3, 159.8

### Example 4

In the same manner as in Example 1, miyabenol A (274.3 mg) was obtained from thunbergii.

### Yellowish brown powder

[α]_{D}=-71.1°(c=0.1 MeOH)
UVλₘₐₓ 322.0, 295.2, 287.2nm(MeOH)
IRνₘₐₓ 3300, 1603, 1520, 1457, 1007cm⁻¹(KBr)
¹H-NMR(400MHz, acetone-d₆)δ : 4.10(1H,d,J=5.8Hz), 4.28(1H,d,J=1.6Hz), 5.07, 4.66(each,1H,d,J=1.9Hz), 5.08(1H,s), 5.09(1H,d,J=5.5Hz), 5.89(2H,d,J=2.1Hz), 6.08(1H,t,J=2.1Hz), 6.60(4H,m), 6.66, 6.32, 6.28, 6.17, 6.05, 5.97(each,2H,d,J=2.1Hz), 6.85, 6.60(each,1H,d,J=1.6Hz), 6.98, 6.79, 6.72, 6.60, 6.56, 6.41 (each,2H,d,J=8.6Hz)
¹³C-NMR(100MHz,acetone-d₆)δ : 50.3, 50.6, 56.0, 56.7, 70.3, 93.8, 95.7, 97.2, 97.6, 103.0, 105.0, 107.8, 108.4, 116.4, 116.5, 116.6, 117.5, 119.7, 120.4, 121.4, 123.1, 127.9, 128.0, 128.4, 128.8, 129.4, 129.8, 132.2, 133.2, 133.5, 134.0, 137.0, 143.5, 147.9, 158.6, 160.1, 160.5, 160.7, 160.9, 162.7, 162.8, 163.5

### Example 5

In the same manner as in Example 1, kobophenol A (50 mg) was obtained from thunbergii.

### Yellowish brown powder

Melting point: 230-235°C
[α]_{D}=+193.4°(c=1.2 MeOH)
UVλₘₐₓ 278.8nm(MeOH)
IRνₘₐₓ 3558-2925, 1615, 829cm⁻¹(KBr)
¹H-NMR(400MHz, acetone -d₆)δ : 3.09(1H,dd,J=10.6,6.2Hz), 3.29(1H,m), 3.48(1H,d,J=3.7Hz), 4.30(1H,d,J=1.8Hz), 4.99(1H,d,J=3.7Hz), 5.03(1H,d,J=4.0Hz), 5.15(1H,d,J=10.6Hz), 5.52(1H,s), 5.80(2H,d,J=2.2Hz), 6.01(3H,m), 6.08(1H,d,J=2.2Hz), 5.95, 6.51(each,1H,d,J=2.2Hz), 5.99, 6.41(each,1H,d,J=2.2Hz), 6.19, 6.49(each,2H,d,J=8.8Hz), 6.39, 6.59(each,2H,d,J=8.8Hz), 6.67, 7.07(each,2H,d,J=8.4Hz), 6.88, 7.33 (each,2H,d,J=8.4Hz)
¹³C-NMR(100MHz,acetone-d₆)δ : 52.1, 52.2, 58.0, 62.0, 84.8, 85.1, 92.4, 93.3, 95.6, 96.1, 102.0, 103.3, 106.6, 109.1, 115.2, 115.7, 115.8, 116.3, 119.8, 119.9, 124.1, 124.1, 126.6, 127.1, 127.4, 128.6, 131.7, 132.2, 133.5, 134.3, 136.2, 139.2, 144.5, 147.4, 156.0, 157.2, 157.6, 158.0, 158.1, 158.6, 159.4, 160.7, 160.9, 161.8

### Example 6

In the same manner as in Example 1, α-viniferin (117 mg, 38 mg, and 33 mg) was obtained from morrowii, foliosissima, and reinii, respectively.

### Yellowish brown powder

Melting point: 213-215°C
[α]_{D}=+24.26° (c=0.9 MeOH)
UVλₘₐₓ 285.6nm(MeOH)
IRνₘₐₓ 3625-3602 1626, 830cm ⁻¹(KBr)
¹H-NMR(400MHz, acetone-d₆)δ : 4.00(1H,s), 4.62(1H,d,J=6.2Hz), 4.71(1H,d,J=6.2Hz), 4.91(1H,d,J=6.2Hz), 5.95(1H,d,J=6.2Hz), 6.00(1H,d,J=1.8Hz), 6.07(1H,s), 6.22(1H,d,J=1.8Hz), 6.24(1H,d,J=2.2Hz), 6.25(1H,d,J=1.83Hz), 6.61(1H,d,J=2.2Hz). 6.72(2H,d,J=8.8Hz), 6.78(2H,d,J=8,8Hz), 6.79(2H,d,J=8.4Hz), 7.03(2H,d,J=8.8Hz), 7.23(2H,d,J=8.4Hz)
¹³C-NMR(100MHz, acetone-d₆)δ : 46.4, 52.8, 55.7, 86.4, 90.0, 95.1, 96.5(2 carbon atom), 96.9, 105.8, 106.2, 108.5, 115.7(2 carbon atom), 116.0(2 carbon atom), 116.1(2 carbon atom), 118.8(2 carbon atom), 119.7, 128.1(2 carbon atom), 128.2(2 carbon atom), 128.7(2 carbon atom), 132.0, 132.3, 132.5, 138.7, 139.7, 141.2, 157.9, 158.2, 158.3, 159.3(2 carbon atom), 159.4, 160.9, 161.6, 161.7

### Example 7

In the same manner as in Example 1, 4,4-(1,3-butadiene-1,4-diyl)bis-phenol (70 mg, 230 mg, and 7.14 mg) was obtained from dispalata, reinii, and ciliato-marginata, respectively.

### Yellowish brown powder

UVλₘₐₓ 272.0nm(MeOH)
¹H-NMR(400MHz, acetone-d₆)δ : 5.80(2H,d,J=12.8Hz). 6.60(2H,d,J=12.8Hz), 6.71(4H,d,J=8.8Hz), 7.54(4H,d,J=8.8Hz)
¹³C-NMR(100MHz, acetone-d₆)δ : 115.8, 128.4, 132.7, 138.9, 139.0, 159.3

### Example 8

Dry powder of sophora of Leguminosae (580 g) was subjected to extraction by use of acetone and methanol in a sequential manner under reflux. The acetone extract was concentrated under reduced pressure, and water was added to the concentrated extract. The resultant mixture was subjected to partition extraction by successive use of benzene, ethyl acetate, and n-butanol. The ethyl acetate extract was concentrated under reduced pressure, to thereby obtain a residue (45 g). The thus-obtained residue was purified through silica gel column chromatography by use of a benzene-acetone solvent, to thereby obtain leachianol A (approximately 200 mg).

### Pale yellow powder

[α]_{D}=-159.8° (c=0.11 MeOH)
UVλₘₐₓ 214, 224, 278nm(MeOH)
IRνₘₐₓ 3300, 1660, 1600cm⁻¹(KBr)
¹H-NMR(400MHz, acetone -d₆)δ : 3.20(1H,d,J=6H2,H-8), 3.52(1H,dd,J=7,1H2,H-12'). 3.70(1H,dd,J=7,6H2,H-7''), 3.78(1H,brd,J=5H2,H-8), 3.81(1H,brs,H-14'), 4.02(1H,brd,J=5H2,H-8'), 4.17(1H,brs,H-7), 4.77(1H,brs,H-7'), 6.24(2H,brs,H-10'',14''), 6.25(1H,t,J=1H2,H-12'), 6.29(2H,d,J=9H2,H-2'',6''), 6.49(1H,d,J=1H2,H-12), 6.54(2H,d,J=9H2,H-3'',5''), 6.69(2H,d,J=8H2,H-3',5'), 6.71(2H,d,J=8H2,H-3,5), 6.77(1H,brs,H-14), 7.03(2H,d,J=8H2,H-2,6), 8.18, 8.25, 8.33, 8.37(×2), 8.51, 8.61(OH)
¹³C-NMR(100MHz, acetone-d₆)δ : 58.0, 48.9, 51.2, 52.3, 53.1, 58.7, 63.6, 72.7, 102.2, 103.1, 103.7, 106.2, 115.8, 116.0, 116.1, 128.9, 157.0, 159.7, 160.1, 168.1, 191.7, 203.6

### Example 9

In the same manner as in Example 8, leachianol B (approximately 600 mg) was obtained.

### Pale yellow powder

[α]_{D}=+147.4° (c=0.25 MeOH)
UVλₘₐₓ 211, 224, 279nm(MeOH)
IRνₘₐₓ 3300, 1760, 1650, 1610cm⁻¹(KBr)
¹H-NMR(400MHz, acetone-d₆)δ : 3.02(1H,brd,J=4H2,H-7''). 3.52(2H,m,H-7,12',8''), 3.78(1H,d,J=7H2,H-8), 4.06(1H,brd,J=7,H-8'), 4.92(1H,brs,H-7'), 5.95(1H,brd,J=7,H-8'), 6.38(1H,d,J=2H2,H-12), 6,68(2H,d,J=8H2,H-2,6), 6,69(1H,brs,H-14' ), 6.73(4H,d,J=8H2,H-3',5,3'',5''),6.85(2H,d,J=8H2,H-3,5), 7.01(4H,d,J=8H2,H-2',6',2'',6''), 8.19, 8.23(×3), 8.26, 8.44(×2) (OH)
¹³C-NMR(100MHz,acetone-d₆)δ : 51.1, 53.6, 56.5, 57.4, 59.4, 70.5, 71.5, 102.2, 102.8, 105.3, 107.8, 115.7, 115.9, 122.2, 126.3, 128.8, 129.0, 133.5, 135.1, 136.9, 141.8, 147.7, 155.1, 156.5, 157.1, 159.1, 159.9, 180.8, 195.5, 201.0

### Example 10

In the same manner as in Example 8, leachianol F (approximately 200 mg) was obtained.

### Brown powder

[α]_{D}=5.9° (c=0.10 MeOH)
UVλₘₐₓ 218, 280nm(MeOH)
IRνₘₐₓ 330, 1610, 1520cm⁻¹(KBr)
¹H-NMR(400MHz, acetone -d₆)δ : 2.95(1H,t,J=4H2,H-8'), 3.37(1H,dd,J=8,4H2,H-8), 4.02(1H,d,J=4H2,C-7-OH), 4.23(1H,d,J=4H2,H-7'), 4.47(1H,dd,J=8,4H2,H-7), 5.92(2H,d,J=2H2,H-10',14'), 6.12(1H,t,J=2H2,H-12'), 6.30(1H,d,J=2H2,H-12), 6.57(1H,d,J=2H2,H-14), 6.68(2H,d,J=8H2,H-3,5), 6.73(2H,d,J=8H2,H-3',5'), 6.85(2H,d,J=8H2,H-2',6'), 6.87(2H,d,J=8H2,H-2,6)
¹³C-NMR(100MHz, acetone-d₆)δ : 55.5, 59.4, 61.7, 76.6, 101.2, 102.4, 106.0, 106.1, 115.4, 115.6, 128.8, 129.2, 136.0, 137.3, 148.6, 155.0, 150.5, 156.2, 156.3, 158.7, 159.1

### Example 11

Dry powder of an underground portion of sophora of Leguminosae (940 g) was subjected to extraction by use of acetone at ambient temperature. The resultant extract was concentrated under reduced pressure, to thereby obtain an acetone extract (50 g). The thus-obtained extract was subjected to elution through silica gel chromatography by use of a benzene-acetone solvent, to thereby obtain davidiol A (approximately 250 mg).

### Brown powder

¹H-NMR(400MHz, acetone-d₆)δ : 2.97(1H,dd,J=12,10H2,H-8c), 4.23(1H,d,J=12H2,H-8b), 4.38(1H,d,J=10H2,H-7c), 4.40(1H,m,H-8a), 5.27(1H,brs,H-7b), 6.02(1H,s,H-12b), 6.08(1H,d,J=3H2,H-7a), 6.19(1H,d,J=3H2,H-12c), 6.42(2H,J=3H2,H-10c,14c), 6.44(1H,brs,H-12a), 6.55(1H,brs,H-14a), 6.60(2H,d,J=9H2), 6.61(2H,d,J=8H2,H-3c,5c). 6.76(2H,d,J=8H2,H-2c,6c), 6.78(2H,d,J=9H2,H-3a,5a), 7.04(2H,d,J=9H2,H-2b,6b), 7.21(2H,d,J=9H2,H-2a,6a), 6.56, 7.93, 7.96(× 2), 7.99, 8.07, 8.30, 8.31(OH)
¹³C-NMR(100MHz, acetone-d₆)δ : 36.5, 50.4, 51.4, 56.2, 67.5, 85.8, 96.0, 101.3, 102.0, 104.0, 108.3, 115.4, 115.6, 116.0, 118.1, 119.2, 128.1, 134.1, 134.4, 137.5, 143.1, 143.9, 147.1, 154.7, 155.8, 156.6, 157.9, 158.1, 158.9, 159.4

### Test Example 1 Measurement of Topoisomerase II Inhibitory Activity

### (A) Extraction of crude topoisomerase II from rat infant brain nuclei (IBN)

IBN (11.98 mg/ml in 50% glycerol) (4.2 µl) was fractionated and subjected to centrifugation (3,000 × g, one minute), to thereby remove glycerol. The thus-obtained pellet was suspended in a nuclear extraction buffer (20 mM Tris-HCl (pH 7.5), 0.3 M NaCl, 140 mM β-Me, 50 ml/ml BSA, 20 mM PMSF) (10 µl), and the suspension was incubated in ice for 30 minutes. Subsequently, the suspension was subjected to centrifugation (10,000 × g, 10 minutes) and supernatant was fractionated, to thereby obtain crude topoisomerase II. The thus-obtained topoisomerase II was diluted on the basis of activity.

### (B) Dilution of purified topoisomerase II (Top GEN, Inc)

Topoisomerase II (Top GEN, Inc) was diluted with the aforementioned nuclear extraction buffer, so as to attain a concentration of 0.75 unit/ml.

A test sample (1 µl) and a buffer (18 µl) containing a mixture of required amounts of Milli Q, 4 × topoisomerase II buffer (250 mM Tris-HCl (pH 7.9), 600 mM KCl, 50 mM MgCl₂, 0.5 mM EDTA, 2.5 mM ATP, 150 mg/ml BSA), and k DNA (312 ng/ml) were added to an Eppendorf tube, and the resultant mixture was vortexed and lightly centrifuged. Subsequently, diluted crude topoisomerase II or diluted purified topoisomerase (1 µl) was added to the mixture, and the mixture was vortexed and lightly centrifuged. (Meanwhile, instead of such topoisomerase II, Milli Q (1 µl) and 50% DMSO (1 µl) were added to a reaction system prepared as a control, which system contains neither enzyme nor xanthone derivative, and 50% DMSO (1 µl) was added to a reaction system containing no xanthone derivative.)

The Eppendorf tube containing crude topoisomerase II and the Eppendorf tube containing purified topoisomerase II were allowed to stand for 30 minutes at 30°C and 37°C, respectively. Subsequently, SDS/PK/BJ (4 µl) was added to both of the tubes in ice, and both were allowed to stand at 50°C for five minutes, vortexed, and lightly centrifuged. After being allowed to stand again at 50°C for 30 minutes, they were vortexed and lightly centrifuged.

### (C) Agarose gel electrophoresis and densitometry

The above-treated mixture was subjected to agarose gel electrophoresis (-EtBr 50 V) by use of a 1 × TBE buffer for electrophoresis (89 mM Tris Base/89 mM boric acid/2 mM EDTA2Na). After electrophoresis, gel staining was performed by use of 1 × TBE (0.1 ml/ml EtBr). After being decolorized, the gel was irradiated with UV rays, and bands on the gel were observed. The concentration of the agarose gel was 0.8%.

The amount of reacted enzyme was quantified by transforming the depth of color of the bands on the gel into numerical values by use of a computer (Gel Plotting Macros installed in NIH Image). Data of bands of mini-circle DNA were processed by a computer for conversion into numerical values and calculation, to thereby obtain residual activity (%). The results are shown in Table 1.

**Table 1**

| Topoisomerase II inhibitory activity | | |
|---|---|---|
| Test sample (Concentration: µg/ml) | | Residual activity (%) |
| Leachianol A | (500) | 2 |
| | (100) | 1 |
| Leachianol B | (500) | 1 |
| | (100) | 1 |
| Mixture of Leachianol F and G | (500) | 1 |
| | (100) | 0 |
| Hopeaphenol | (500) | 1 |
| | (100) | 4 |
| Davidiol A | (500) | 2 |
| | (100) | 3 |
| 4,4-(1,3-Butadiene-1,4-diyl)bis-phenol | (500) | 0 |
| | (100) | 28 |
| Miyabenol A | (10) | 0 |
| | (1) | 13 |
| | (0.5) | 32 |
| Pallidol | (50) | 0 |
| | (2) | 3 |
| | (1) | 30 |
| Miyabenol C | (10) | 0 |
| | (2) | 42 |
| | (0.5) | 57 |
| α-Viniferin | (50) | 0 |
| | (1) | 38 |
| | (0.5) | 41 |
| Kobophenol A | (50) | 18 |
| | (10) | 59 |
| | (0.5) | 68 |

As is apparent from Table 1, the active ingredients of the present invention exhibit topoisomerase II inhibitory activity.

### Industrial Applicability

The active ingredients of the present invention exhibit excellent topoisomerase II inhibitory activity and are effective as antitumor agents.

## Claims

1. An antitumor agent comprising, as an active ingredient, a compound selected from among pallidol, miyabenol A, miyabenol C, α-viniferin, kobophenol A, 4,4-(1,3-butadiene-1,4-diyl)bis-phenol, leachianol A, leachianol B, leachianol F, leachianol G, hopeaphenol, and davidiol A, or a salt thereof.

2. An antitumor agent composition comprising a compound selected from among pallidol, miyabenol A, miyabenol C, α-viniferin, kobophenol A, 4,4-(1,3-butadiene-1,4-diyl)bis-phenol, leachianol A, leachianol B, leachianol F, leachianol G, hopeaphenol, and davidiol A, or a salt thereof, and a pharmaceutically acceptable carrier.

3. Use of a compound selected from among pallidol, miyabenol A, miyabenol C, α-viniferin, kobophenol A, 4,4-(1,3-butadiene-1,4-diyl)bis-phenol, leachianol A, leachianol B, leachianol F, leachianol G, hopeaphenol, and davidiol A, or a salt thereof for producing an antitumor agent.

4. A method for treating tumors, which comprises administering a compound selected from among pallidol, miyabenol A, miyabenol C, α-viniferin, kobophenol A, 4,4-(1,3-butadiene-1,4-diyl)bis-phenol, leachianol A, leachianol B, leachianol F, leachianol G, hopeaphenol, and davidiol A, or a salt thereof to a patient in need.
